# EUROPEAN PATENT APPLICATION

(11) **EP 1 593 383 A1**
(43) Date of publication of application: **09.11.2005**
(21) Application number: 04101884.7
(22) Date of filing: 03.05.2004
(51) Int. Cl.: A61K 35/78, A61P 37/08

(54) **Lotus plant extract with antihistaminic activity**

(71) Applicant: ROYAL VETERINARY & AGRICULTURAL UNIVERSITY, DK-1871 Frederiksberg C, Copenhagen (DK)
(72) Inventor: Bak, Soren, 2200 Copenhagen N (DK); Forslund, Karin, 75325, Uppsala (SE); Moeller, Birger, 2700, Broenshoej (DK)
(74) Representative: Markvardsen, Peter

(57) **Abstract**

An extract from a plant of the genus *Lotus,* in particular of *Lotus japonicus,* comprising rhodiocyanoside A and rhodiocyanoside D, a process for preparing it, a pharmaceutical composition comprising it and uses thereof for the manufacture of a medicament with antihistaminic activity.

## Description

### FIELD OF THE INVENTION:

The present invention relates to an extract from a plant of the genus *Lotus*, in particular of *Lotus japonicus*, comprising rhodiocyanoside A and rhodiocyanoside D, a process for preparing it, a pharmaceutical composition comprising it and uses thereof for the manufacture of a medicament with anti-histaminic activity.

### BACKGROUND OF THE INVENTION:

Rhodiocyanoside A and rhodiocyanoside D are two nitrile glucosides, which have been assignated the CAS registry numbers 168433-86-1 and 197508-56-8, respectively. The chemical name of rhodiocyanoside A is (Z)-4-(β-D-glucopyranosyloxy)-2-methyl-2-butenenitrile. The chemical name of rhodiocyanoside D is (E)-2-[(β-D-glucopyranosyloxy)methyl]-2-butenenitrile. Both show interesting anti allergic activity and have been found in a few plant species.

Rhodiocyanoside A was first isolated from the Chinese natural medicine "Si Lie Hong Jing Tian" (Shiretsukoukeiten in Japanese) and its structure was elucidated. Rhodiocyanoside A exhibited inhibitory activity on the histamine release and was found to inhibit the passive cutaneous anaphylaxis reaction. (Yoshikawa M, et al. Chem Pharm Bull (Tokyo) (1995) 43: 1245-1247.)

The Chinese Natural Medicine Si-Li-Hong-Jing-Tian is produced from the underground part of Rhodiola quadrifida (Pall) Fisch Et Mey and it has been used as a hemostatic, an antibechnic and an endermic liniment for burns.

Rhodiocyanoside D was first isolated from the methanolic extract of the underground part of Rhodiola sacra (Prain ex Hamet) S.H. Fu (Crassulaceae) and its chemical structure was determined. All the major chemical constituents from R.sacra inhibited the histamine release (Yoshikawa M, et al. Chem Pharm Bull (Tokyo) (1997) 45: 1498-1503).

Rhodiocyanoside A was also isolated from the methanolic extract of the underground part of Rhodiola sachalinensis (Chem. Pharm. Bull. (2001), 49(4), 396-401).

### SUMMARY OF THE INVENTION:

The problem to be solved by the present invention is to provide alternative plant comprising rhodiocyanoside A and rhodiocyanoside D, from which one could obtain extracts comprising said rhodiocyanosides.

As explained above, extracts comprising rhodiocyanoside A and rhodiocyanoside D described in the prior art have been obtained from plants of the genus Rhodiola, and in particular from the roots of these plants.

Surprisingly, the present inventors have found that plants not only of a different genus, but of a different order provide extracts particularly enriched in rhodiocyanosides A and D.

The solution is based on plants of the genus Lotus.

Advantageously, plants of the genus Lotus provide plant material from which extracts specially enriched in rhodiocyanosides A and D can be obtained. Extracts from Lotus japonicus are particularly advantageous, since have been found to contain amounts of said active compounds significantly superior than those extracts obtained from Rhodiola plants described in the prior art. Surprisingly, said compounds were not detected in the root extracts, but extracts from the seedlings and the apical leaves contained high content of said active compounds.

Accordingly, a first aspect of the invention relates to an extract from a plant of the genus *Lotus* comprising rhodiocyanoside A and rhodiocyanoside D.

A second aspect of the invention relates to a process for preparing an extract as described herein, comprising:
a) Extracting the plant of the genus Lotus as described herein or parts thereof with an extraction agent comprising a water miscible organic solvent or a mixture thereof with water and
b) Subsequently removing said extracting agent.

A third aspect of the invention relates to a composition comprising rhodiocyanoside A and/or rhodiocyanoside D obtained by a process of claim 6 and wherein the composition comprises some impurities from the plant of the genus Lotus used in the extraction process.

A fourth aspect of the invention relates to a pharmaceutical composition comprising an extract according to the invention or a composition comprising rhodiocyanoside A and/or rhodiocyanoside D according to the third aspect of the invention and a pharmaceutically acceptable carrier.

Still a further aspect of the invention relates to a use of an extract as described herein or a composition comprising rhodiocyanoside A and/or rhodiocyanoside D according to the third aspect of the invention, for the manufacture of a medicament with anti-histaminic activity and for the manufacture of a medicament for the treatment or prophylaxis of a disease selected from allergic conditions, asthma, hypersensitivity.

Embodiments of the present invention are described below, by way of examples only.

### BRIEF DESCRIPTION OF THE DRAWINGS

Figure 1 shows the results of the analysis by LC-MS-SIM of cyanogenic glucosides and nitrile glucosides in shoots and roots of *Lotus japonicus*. Total ion chromatogram (TIC) and extracted ion chromatogram (EIC) of specific [M+ Na]⁺ adducts are presented.
Figure 2 shows the relative percentage content of the cyanogenic- and nitrile glucosides in *L. japonicus* tissues.
Figure 3 shows the complete biosynthesis pathway for making the Rhodiocyanoside A and D in *Lotus japonicus*.
Figure 4 shows the total amount of the four glucosides; linamarin, lotaustralin, rhodiocyanoside A and rhodiocyanoside D, in shoots from the transgenic line # 5 of *Lotus japonicus*

### DETAILED DESCRIPTION OF THE INVENTION:

### Plant of the genus Lotus

In principle the plant may be any plant of the genus *Lotus*. Now that the present inventors have disclosed that plants of the genus Lotus comprise rhodiocyanosides A and D it is routine work for the skilled person to identify a specific Lotus plant of interest that comprise the rhodiocyanosides.

One may for instance take a number of different known plants of the genus Lotus, making extracts of these and analyze the extract for presence of rhodiocyanosides A and D. The analysis for presence of rhodiocyanosides A and D may rapidly and routinely e.g. be done by use of a Liquid Chromatography - Mass spectrometry (LC-MS) based protocol as outlined in working examples herein.

Preferably, the plant is *Lotus japonicus*.
Plant of the specie *Lotus japonicus* are well known in the art. Reference is made to (Larsen K, Botanisk Tidsskrift 1955, 52, 8-17) for a general description of *Lotus japonicus*.

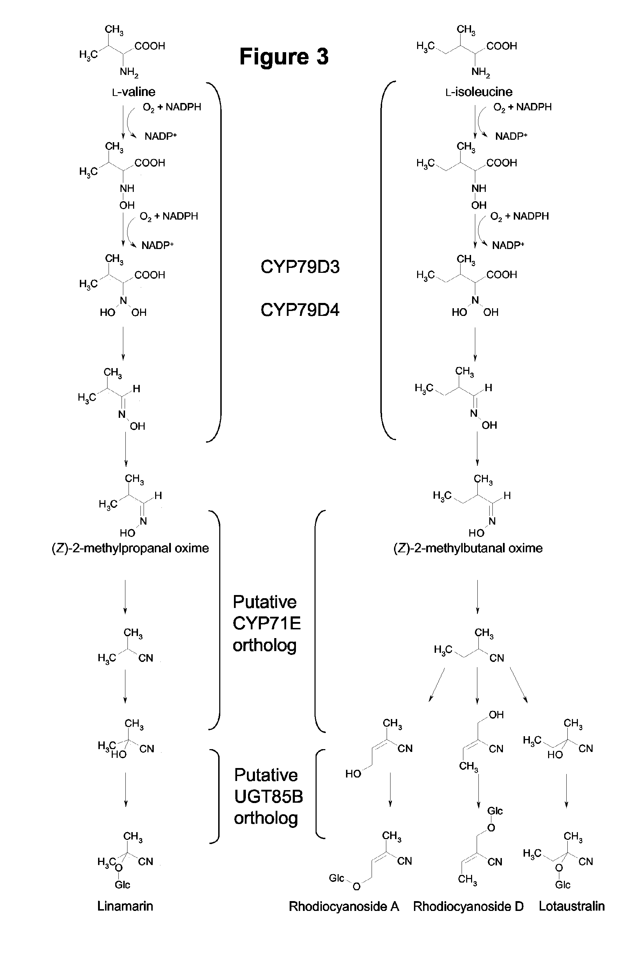

Figure 3 shows the complete biosynthesis pathway for making the Rhodiocyanoside A and D in *Lotus japonicus* and in working examples herein is made a transgenis Lotus plant. Accordingly, the term "plant of the genus *Lotus*" shall herein also be understood to emcompass transgenic plants.

### Extraction of the plant or parts thereof

Extracts according to the invention are obtained by extraction of a plant material derived from a whole plant or parts thereof. Parts of the plant include e.g. leaves, shoot tips, seeds, seedlings, stems, flowers, roots.

The plant material may be e.g. fresh, frozen or dried.

In Lotus japonicus, the parts of the plant preferred are leaves and seedlings. More preferably, shoot tips.

### Culture

The plant may be cultured conventionally, namely, in the soil, outdoors or in a greenhouse, or, alternatively, in a soil-free culture such as e.g. a hydroponically culture.

### Extraction agent comprising a miscible organic solvent

The first step of the extraction process comprises the extraction of the plant with an extraction agent comprising a water miscible organic solvent or a mixture thereof with water. The extraction may be performed with a number of different water miscible organic solvents and mixtures thereof with water. Examples of preferred extraction solvents include acetone, methyl ethyl ketone, ethyl acetate, lower alkanols having 1 to 4 carbon atoms and mixtures thereof with water. The preferred extraction solvents are lower alkanols miscible in water having 1 to 4 carbon atoms, such as methanol, ethanol, isopropylic alcohol, propanol, *tert*-butyl alcohol.

The extraction can be performed hot or cold by the employment of any extraction technology e.g. maceration, percolation or supercritical extraction. The preferred extraction temperature is close to the boiling point of the employed solvent due to extraction efficacy, but lower temperatures are also applicable making necessary a longer period of extraction.

### Removing of the extracting agent

After the primary extraction process a second step of removing the extraction agent is performed. Different techniques may be employed to remove or to concentrate the extract, such as liquid-liquid extraction, column chromatography, steam distillation, vacuum distillation or lyophilization. Preferably the extraction agent is removed by lyophilization.

Preferably, is the extract comprising a total amount of the two glucosides rhodiocyanoside A and rhodiocyanoside D of at least 0.50% of the dry weight, more preferably is the extract comprising a total amount of the two glucosides rhodiocyanoside A and rhodiocyanoside D of at least 1% of the dry weight and even more preferably is the extract comprising a total amount of the two glucosides rhodiocyanoside A and rhodiocyanoside D of at least 1.25% of the dry weight.

The extract may also comprise the two glucosides linamarin and lotaustralin.

### Dissolution of the residue

In a preferred embodiment, after the second step of removing the extraction agent, the residue obtained is dissolved in water and subsequently extracted with a solvent immiscible in water.

### Extraction with a solvent inmiscible in water

The solvent immiscible in water is preferably a linear, ramified or cyclic aliphatic hydrocarbon, having from 5 to 8 carbon atoms, such as *n*-pentane, *n*-hexane, *n*-heptane, cyclohexane. Preferably *n*-pentane, *n*-hexane or *n*-heptane. More preferably *n*-pentane.

### Suitable subsequent steps

Regardless of the mode of preparation according to the invention, subsequent steps intended to promote preservation and/or stabilization may be included without as a result modifying the actual nature of the extract. Thus, for example, the extract obtained may be freeze-dried by any conventional freeze-drying method. A powder is thus obtained which may be used directly or, alternatively, mixed in an appropriate solvent before use.

### Further purification of the rhodiocyanoside A and/or rhodiocyanoside D

Optionally, the extract may be further purified in order to isolate rhodiocyanoside A and/or rhodiocyanoside D with the desired degree of purity. Different techniques known in the art may be employed, such as chromatographic techniques or liquid-liquid extraction.

The skilled person knows how to do that and depending on the specific needs of interest a composition comprising at least 75% rhodiocyanoside A and/or rhodiocyanoside D, at least 85% rhodiocyanoside A and/or rhodiocyanoside D or at least 99% rhodiocyanoside A and/or rhodiocyanoside D may be obtained.

The extra purification may e.g. be by LC-MS as described in working examples herein, where the rhodiocyanoside A and/or rhodiocyanoside D are purified by isolation of the peaks corresponding to these.

Preferably, the composition comprises both rhodiocyanoside A and rhodiocyanoside D.

### Extracts

Preferably, the extracts of the present invention are enriched on rhodiocyanosides A and/or D.

Preferably, the extracts of the present invention have a low content of hydrolyzing enzymes, in particular of beta-glucosidases, more preferably they are not present. These enzymes may be removed or its content diminished by techniques known in the art, e.g. by introducing an additional step of purification of the extract the extract (by e.g. centrifugation, ultrafiltration).

Alternatively these enzymes in the extract may be inactivated. An enzyme's activity can be disrupted through any chemical, thermal or physical method that alters the tertiary configuration of the protein. The three broad classes of inactivation are inhibition, denaturation and destruction. Inhibition is usually accomplished by a change in pH or ion balance, or by the introduction of enzyme-specific inhibition proteins; it results in a reversible or irreversible blockage of access to and/or shape-change of the enzyme's active site. Denaturation - a partial or complete unraveling of the protein's tertiary structure - is usually accomplished through extreme pH or heat, simple drying, solvents (such as ethanol or acetone), or surfactants (e.g. sodium dodecylsulfate, SDS); the denaturation may be partially or fully reversible if the denaturant is removed or neutralized and if the denaturation is not too far progressed. Irreversible destruction involves chemically changing the nature of the protein; in conservation this would typically be achieved by permitting the air to oxidize a dried protein residue or by cleavage with a protease.

The conservation literature has conventionally prescribed the denaturation of enzymes with heated water or ethanol.

A composition comprising rhodiocyanoside A and/or rhodiocyanoside D and impurities from the plant of the genus Lotus:

An aspect of the invention relates to a composition comprising rhodiocyanoside A and/or rhodiocyanoside D obtained by a process for making such a composition as described herein and wherein the composition comprises some impurities from the plant of the genus Lotus used in the extraction process.

The term "obtained from" shall herein be interpreted restrictive in the sense that one has used a plant of the genus Lotus as a starting material to make the composition comprising rhodiocyanoside A and/or rhodiocyanoside D.

The term "impurities" means any impurity (e.g. another compound, molecule or other element than rhodiocyanoside A and/or rhodiocyanoside D) which originate from the plant of the genus Lotus originally used to make first the extract and then by further purification composition comprising rhodiocyanoside A and/or rhodiocyanoside D.

Depending on the degree of purification such impurities may be in greater or smaller amounts. It may be that the composition comprises less than 50% of such impurities, less than 10% of such impurities or less than 0.5% of such impurities.

Preferably, the composition comprises both rhodiocyanoside A and rhodiocyanoside D.

### Antihistaminic activity

The inhibitory effect of rhodiocyanosides A and D on histamine release is known, as described above.

In the present invention, by a medicament with antihistaminic activity it is understood a medicament that has the action of curing and relieving the pathophysiological symptoms due to the release of histamine.

Histamine is a potent mediator of numerous biological reactions. In the human organism it is virtually ubiquitous in tissues and body fluids, being mainly stored in its inactive form in the metachromatic granula of mast cells and basophilic leukocytes. Following stimulation of mastcells and basophils, histamine is released explosively into the surrounding tissues and bodyfluids. On release, histamine functions as a potent mediator of numerous physiological and pathophysiological processes in nearly all organs and tissues. A possible role for histamine in immunological and inflammatory reactions has been concluded from its interactions with various cytokines. Histamine has been clearly implicated as a primary mediator of "acute phase" allergic reactions and hypersensitivity reactions. Its biological activities in tissue are mediated by three different cell surface receptors (i.e. H1, H2 and H3 receptors).

In asthma, histamine interacts with bronchial smooth muscle cells and other cells, leading to bronchial constriction.

Therefore compositions and extracts according to the invention, comprising the antihistaminics rhodiocyanosides A and/or D, may be useful in the treatment or prophylaxis of allergic conditions, asthma, hypersensitivity.

Allergic conditions include, but are not limited to, "immediate type" allergies such as hay fever, allergic asthma, urticaria, neurodermatitis and anaphylaxis; allergy to the consumption of different foods; allergic intolerance to drugs, paints or cosmetics as well as various physical stimuli such as light, contact, heat or cold, stress or painful situations.

### Pharmaceutically acceptable carrier

By "pharmaceutically acceptable carrier" as used herein is meant one or more compatible solid or liquid filler diluents, or encapsulating substances. By "compatible" as used herein is meant that the components of the composition are capable of being commingled without interacting in a manner which would substantially decrease the pharmaceutical efficacy of the total composition under ordinary use situations.

Some examples of substances which can serve as pharmaceutical carriers are sugars, such as lactose, glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; vegetable oils, such as peanut oils, cotton seed oil, sesame oil, olive oil, corn oil and oil of theobroma; polyols such as propylene glycol, glycerine, sorbitol, manitol, and polyethylene glycol; agar; alginic acids; water; pyrogen-free water; isotonic saline; and phosphate buffer solution; skim milk powder; as well as other non-toxic compatible substances used in pharmaceutical formulations.

Wetting agents, fillers and lubricants such as sodium lauryl sulfate, as well as coloring agents, flavoring agents, lubricants, excipients, tabletting agents, stabilizers, anti-oxidants and preservatives, can also be present.

A pharmaceutical composition as described herein can be administered orally, intramuscularly, intravenously, transdermally, subcutaneously or by other modes of administration. Preferably, the pharmaceutical product can be administered orally.

A pharmaceutical composition, as described herein, may include other pharmaceutically active substances. It can be prepared by mixing the active compounds with one or more pharmacologically tolerated auxiliaries and/or excipients such as, for example, fillers, emulsifiers, lubricants, masking flavors, colorants, or buffer substances, and converting the mixture into a suitable pharmaceutical form such as, for example, tablets, coated tablets, capsules, granules, powders, emulsions, suspensions, or solutions.

Examples of auxiliaries and/or excipients which may be mentioned are tragacanth, lactose, talc, agar, polyglycols, ethanol, and water. It is also possible to administer the active substances as such, without vehicles or diluents, in a suitable form, for example, in capsules.

Preferably, the pharmaceutical product, as described herein, is provided together with suitable pharmaceutically relevant instructions. The instructions preferably explain pharmaceutically relevant information such as e.g. qualitative and quantitative of composition, pharmaceutical form, therapeutic indications and method of administration (including recommend doses).

### EXAMPLES:

### Example 1: Process for preparing a Lotus extract

### Culture of plants

Seeds from *Lotus japonicus* GIFU B-129-S9 were scarified by a short treatment with sand paper and surface sterilised in 2 % hypochlorite with 0.02 % Tween (shaking, 20 min). The seeds were washed in 6 shifts of autoclaved water and germinated for 1 week on water saturated filter paper (20°C, photosynthetic flux of 100-120 µmol photons m⁻² s⁻¹, and a light dark regime of 16/8 h). Alternatively, one may start from a suitable part of the plant such as shoot.
All plant material was grown hydroponically with the seedlings mounted with foam in Eppendorf tubes from which the bottom had been cut off. The Eppendorf tubes were then mounted in the lid of a 4 L-high density polyethylene container filled with aerated nutrient solution (Husted et al., 2002), and placed in a greenhouse at 24°C fitted with extra light bulbs ensuring a minimum photosynthetic flux of 100-120 µmol photons m⁻² s⁻¹ and a 16/8 light dark regime. The nutrient solution was changed once a week. Plant tissue used for the different analyses was harvested into liquid nitrogen and stored at -80° until use.

Preparation of extract *L. japonicus* plant material was extracted in 85 % (v/v) boiling methanol. The solvent was evaporated by lyophilization and the residue dissolved in water and extracted three times with *n*-pentane. The rhodiocyanosides and cyanogenic glucosides are recovered from the aqueous phase and further purified by preparative LC-MS or TLC or other standard chromatographic techniques.

### Example 2: Analysis of the extract by LC-MS-SIM

### Introduction

The objective of this example was to analyze the presence and absence of cyanogenic glucosides and nitrile glucosides (e.g. rhodiocyanoside A and rhodiocyanoside D) in various *L. japonicus* tissues by LC-MS-SIM.

Nitrile glucosides have in a few cases been shown to co-occur with cyanogenic glucosides (Lechtenberg *et al*., *Phytochemistry* (1996) 41: 779-785; Nielsen *et al*., *Plant Physiol* (2002) 129: 1066-1075). Cyanogenic glucosides result in concomitant release of toxic hydrogen cyanide, glucose and an aldehyde or ketone when degraded by beta-glucosidases and alfa-hydroxynitrilases. As cyanogenic glucosides, nitrile glucosides are β-glucosides of hydroxy nitriles. However, in nitrile glucosides (e.g. rhodiocyanoside A and rhodiocyanoside D), the hydroxyl and nitrile groups are not linked to the same carbon atom of the aglycone. Accordingly, hydrolysis of nitrile glucosides by β-glucosidases does not result in HCN release.

This example shows that *Lotus japonicus* contains the two nitrile glucosides rhodiocyanoside A and rhodiocyanoside D as well as the cyanogenic glucosides linamarin and lotaustralin.

### Materials and methods

Aliquots of the aqueous phase of the extract of example 1 were subjected to LC-MS-SIM analysis using a HP1100 LC coupled to a Bruker Esquire-LC ion trap mass spectrometer (Bruker Instruments, Billerica, MA). A Waters Xterra MS C18 column (Waters Milford, MA) was used and the LC conditions were as described in (Nielsen et al., *Plant Physiol* (2002) 129: 1066-1075) . The mass spectrometer was run in positive ion mode. The results were analysed by Bruker Daltonics DataAnalysis version 3.0.

Linamarin was verified based on identical mass, retention times, and fragmentation pattern compared to an authentic standard. Lotaustralin, rhodiocyanoside A and D were isolated by preparative LC and their identity verified by NMR-spectrometry.

### Results

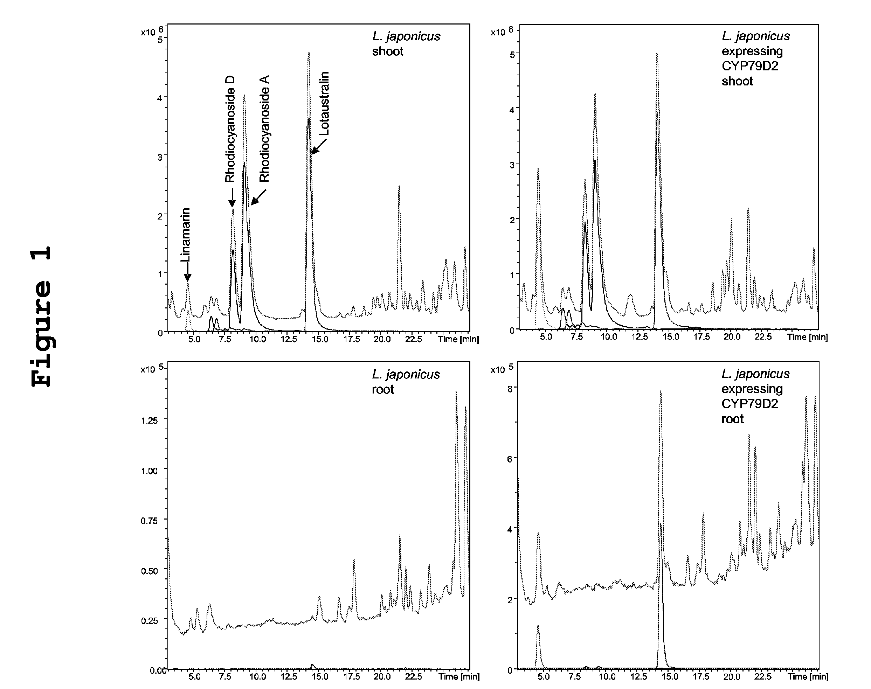

Figure 1 shows the results of the analysis by LC-MS-SIM of cyanogenic glucosides and nitrile glucosides in shoots and roots of *Lotus japonicus*. Total ion chromatogram (TIC) and extracted ion chromatogram (EIC) of specific [M+ Na]⁺ adducts are presented. Linamarin (EIC, 270, rt=4.5 min), rhodiocyanoside D (EIC 282 rt=8.5 min), rhodiocyanoside A (EIC, 282 rt=9.5 min), and lotaustralin (EIC 284, rt=14.3 min). To visualize the minute amounts of cyanogenic glucosides and nitrile glucosides in roots, different scales are applied for shoots and roots.

### Discussion

Glucosides readily form adducts with sodium ions. Sodium adducts of the cyanogenic glucosides linamarin and lotaustralin and of the nitrile glucosides, rhodiocyanoside D and rhodiocyanoside A were detected, albeit preferentially in aerial tissues (Fig. 1). The identity of the compounds was confirmed by NMR spectrometry (data not shown). In roots, minute amounts of lotaustralin were occasionally detected.

### Example 3: Content of rhodiocyanosides in different tissues of Lotus japonicus

This example shows that the content of cyanogenic- and nitrile glucosides in *L. japonicus* depends on plant developmental stage and tissue. The cyanide potential is highest in young seedlings and in apical leaves of mature plants, while the presence of the nitrile glucoside rhodiocyanoside A is higher in apical leaves.

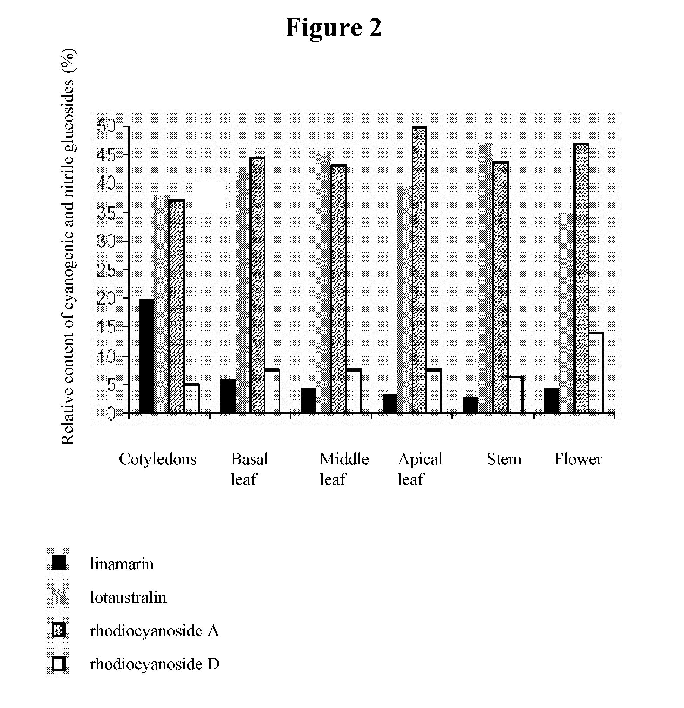

Figure 2 shows the percentage content of the cyanogenic- and nitrile glucosides in *Lotus japonicus* tissues. Except for flowers, the tissues were obtained from 18-days-old hydroponically grown plants (see example 1). Quantification is based on peak areas from LC-MS-SIM chromatograms.

### Discussion

In *L. japonicus* aerial tissues, rhodiocyanoside A and lotaustralin are two major components, while linamarin and rhodiocyanoside D are minor components (Fig. 2). These four constituents co-occur in the different aerial tissues of *L. japonicus*, and the ratio between the components is relatively constant, except in cotyledons where the proportion of linamarin is higher (Fig. 2).

### Example 4: Isolation of rhodiocyanosides A and D

Rhodiocyanosides A and D were isolated from the extracts obtained as described in example 1.

Total Rhodiocyanoside A + D content:
FW: fresh weight
- 4-days-old seedlings: 10nmoles per mg FW
Approximately 2500 ng = 2.5 microg = 2.5 g per kg FW
- Young leaves/ shoot tips
Approximately 6 nmoles per mg FW = 1500 ng/mg FW = 1.5 g/kg FW

### Example 5: Transgenic L. japonicus expressing Cassava CYP79D2

The cassava *CYP79D2* cDNA containing the 35S promoter and polyadenylation site was excised from pPZP221 cauliflower mosaic virus *35S::CYP79D2* constructs (Mikkelsen MD, Halkier BA (2003), Plant Physiol 131: 773-779) using *Hind*III and ligated into the corresponding *Hind*III site in pPZP111 (Hajdukiewicz P, Svab Z, Maliga P (1994), Plant Mol Biol 25: 989-994) to confer resistance to G-418 DISULPHATE, and the final construct was introduced into *Agrobacterium tumefaciens* by electroporation (Shen WJ, Forde BG (1989), Nucl Acids Res 17(20): 8385). *L. japonicus* was transformed using hypocotyls (Handberg K, Stougaard J (1992), Plant J 2: 487-496). The explants were grown at 25°C with light dark regime of 16/8 h. Transformants were selected using the gentamycin analogue G-418 (25 mg l⁻¹) and tested for expression of the neomycin phosphotransferase NPT II protein using the NPT II ELISA kit (Patho Screen NPTII, Agdia). Independent transgenic lines were selected as evidenced by the expression of NPTII product and segregation analysis on G-418 disulphate. Cuttings from the *in vitro* grown transformants were grown hydroponically as described above.

Twenty-two individual transformants were selected and the ratio between linamarin and lotaustralin was determined by LC-MS-SIM. Of the 22 lines tested, the line designated 35S::*CYP79D2*#5 was selected for further analysis as it contained a single insert as monitored by segregation analysis, and because it contained the highest ratio of linamarin to lotaustralin content. The line appeared morphometrically indistinguishable from wild type. In line 35S::*CYP79D2*#5, the cyanide potential was 16 nmol cyanide/Fw (mg)⁻¹, approximately twice as high as in wild type plants. While the linamarin content was increased ~20 fold, the lotaustralin content was only slightly increased. Accordingly, the increase in cyanide potential is mainly the consequence of increased linamarin content. The ratio of rhodiocyanoside A and D to lotaustralin was unaltered in leaves (Figure 1). In roots expressing cassava *CYP79D2*, linamarin and lotaustralin could be detected although in much smaller quantities than in green tissue (Figure 1). In wild type roots, the level of lotaustralin and especially linamarin was very low and in most cases below detection limit (Figure 1).

### Example 6: Comparing the contents of rhodiocyanosides A and D, linamarin and lotaustralin in Lotus japonicus with herbal tablets made from Rhodiola species.

Two kinds of commercially available herbal tablets were extracted and compared to extracts of *Lotus japonicus* shoots from the transgenic line # 5 (see example above) by metabolite profiling (LC-MS-MS). Plant material as well as milled tablets were extracted in boiling 85 % methanol. These extracts were evaporated to a minimum, redissolved in water and extracted three times with n-pentane to e.g. remove lipids and chlorophyll. After filtering and cold evaporation the extracts were analyzed by LC-MS-MS.

### The tablets were:

*Rhodiola Rosea* from *Winther Medico A*/*S* prepared from extracts of the roots of *Rhodiola Rosea.*

*Rhodiola Plus* from *HerbMax, U.S. Pharmatec* prepared from *Rhodiola Sacra* and *Gingko Biloba.*

Plants of the *Rhodiola Rosea* species have not yet been proved to contain the four glucosides of interest, whereas the rhodiocyanosides and lotaustralin were previously isolated from the roots of a couple *of Rhodiola Sacra* species.

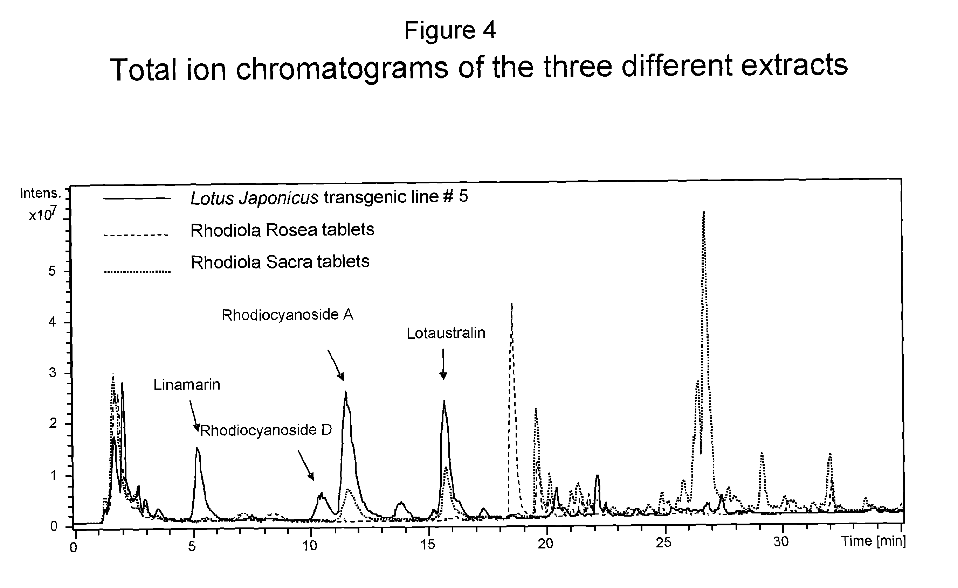

The total amount of the four glucosides; linamarin, lotaustralin, rhodiocyanoside A and rhodiocyanoside D, in shoots from the transgenic line # 5 of *Lotus japonicus* is approximately 3 % of the dry weight and linamarin makes up about 25 % of this. Based on this approximation and calculated areas under the peaks in the LC-MS-chromatograms (see figure 4) the content of the relevant glucosides in the *Rhodiola Rosea* tablets was determined to be approximately 0,2 % of the tablets' mass, none of which was linamarin. *Rhodiola Plus* apparently did not contain linamarin, lotaustralin, rhodiocyanoside A and rhodiocyanoside D.

The sums of areas of all peaks (between t_{R} 3 and 35 minutes) in the *Rhodiola Rosea* and *Lotus japonicus* chromatograms were also calculated. The contents of the four nitril-glucosides in the *Lotus* extract made up 35 % of the total amount of compounds recovered in the above described extraction procedure, whereas the relative contents of linamarin, lotaustralin, rhodiocyanoside A and rhodiocyanoside D, in the tablet extract was about tenfold less.

All in all, this means that the *Lotus japonicus* shoots are a more potent source of linamarin, lotaustralin, rhodiocyanoside A and rhodiocyanoside D, than are the tablets. Furthermore the shoots apparently give a "cleaner" extract with respect to these compounds than the *Rhodiola* species.

Similar results were also obtained in extracts obtained from wildtype *Lotus japonicus* plant shoots.

## Claims

1. An extract obtained from a plant of the genus *Lotus* comprising rhodiocyanoside A and rhodiocyanoside D.

2. The extract according to claim 1, wherein said plant is *Lotus japonicus*.

3. A process for preparing an extract according to any of the preceding claims, comprising:
a) Extracting the plant or parts thereof of claims 1 or 2 with an extraction agent comprising a water miscible organic solvent or a mixture thereof with water and
b) Subsequently removing said extracting agent.

4. The process according to claim 3, further comprising the steps of:
c) Dissolving the residue obtained in step b) in water and
d) Subsequently extracting with a solvent immiscible in water.

5. The process according to any of claims 3 to 4, wherein said solvent of step a) is a C1-C4 alkanol.

6. The process according to any of claims 3 to 5 further comprising the step of isolating the rhodiocyanoside A and/or rhodiocyanoside D in a suitable way to get a composition comprising rhodiocyanoside A and/or rhodiocyanoside D with a desired degree of rhodiocyanoside A and/or rhodiocyanoside D purity.

7. A composition comprising rhodiocyanoside A and/or rhodiocyanoside D obtained by a process of claim 6 and wherein the composition comprises some impurities from the plant of the genus Lotus used in the extraction process.

8. A pharmaceutical composition comprising an extract according to any of claims 1 to 2 or a composition comprising rhodiocyanoside A and/or rhodiocyanoside D of claim 7 and a pharmaceutically acceptable carrier.

9. Use of an extract according to any of claims 1 to 2 or a composition comprising rhodiocyanoside A and/or rhodiocyanoside D of claim 7, for the manufacture of a medicament with anti-histaminic activity.

10. Use of an extract according to any of claims 1 to 2 or a composition comprising rhodiocyanoside A and/or rhodiocyanoside D of claim 7, for the manufacture of a medicament for the treatment or prophylaxis of a disease selected from allergic conditions, asthma, hypersensitivity.
